# FASCICULE DE BREVET EUROPEEN

(11) **EP 0 770 376 B1**
(45) Date de publication et mention de la délivrance du brevet: **10.12.1997**
(21) Numéro de dépôt: 96402112.5
(22) Date de dépôt: 03.10.1996
(51) Int. Cl.: A61K 7/32

(54) **Utilisation d'un antagoniste de substance P dans une composition topique comme agent antitranspirant**
Verwendung eines Antagonisten der Substanz P in einer topischen Zusammensetzung als Antitranspirationsmittel
Use of a substance P antagonist in a topical composition as antiperspirant

(30) Priorité: 26.10.1995 FR 9512655
(43) Date de publication de la demande: 02.05.1997
(73) Titulaire: L'OREAL, 75008 Paris (FR)
(72) Inventeur: de Lacharriere, Olivier, 75015 Paris (FR); Breton, Lionel, 78000 Versailles (FR)
(74) Mandataire: Lhoste, Catherine

(56) Documents cités:
- EP-A- 0 522 808
- US-A- 4 472 305

## Description

La présente invention concerne l'utilisation d'un antagoniste de substance P dans une composition cosmétique et/ou dermatologique agent antitranspirant.

La substance P est un élément chimique polypeptidique élaboré et libéré par une terminaison nerveuse. Elle intervient notamment dans la transmission de la douleur et dans des maladies du système nerveux central telles que l'anxiété, la schizophrénie, dans des maladies respiratoires et inflammatoires, dans des maladies gastrointestinales, dans des maladies rhumatismales et dans certaines maladies dermatologiques.

Par la demande FR-94/05537 du 5 mai 1994 déposée au nom du demandeur, il est connu d'utiliser des antagonistes de substance P empêchant la synthèse et/ou la libération et/ou la fixation de la substance P pour traiter les peaux sensibles. Toutefois, ce document n'enseigne pas que ces antagonistes de substance P peuvent être utilisés comme agent antitranspirant.

Les agents antitranspirants généralement utilisés comme par exemple les sels d'aluminium (chlorohydrate d'aluminium, complexe d'aluminium et de zirconium) présentent l'inconvénient d'être irritants et d'être mal supportés notamment par les personnes à peau sensible. En particulier, ces dernières ressentent des démangeaisons, des picotements, des échauffements, des tiraillements, des fourmillements et/ou des rougeurs. Ces signes s'associent souvent à des érythèmes.

Il subsiste donc le besoin d'une composition topique antitranspirante non irritante, utilisable notamment par les personnes à peau sensible et présentant une efficacité équivalente, voire supérieure à celles de l'art antérieur.

La présente invention a donc pour objet l'utilisation d'au moins un antagoniste de substance P dans ou pour la préparation d'une composition topique comme agent transpirant. Elle se rapporte plus particulièrement à l'utilisation d'au moins un antagoniste de substance P, dans ou pour la préparation d'une composition cosmétique ou dermatologique, pour prévenir et/ou lutter contre la transpiration cutanée, chez l'être humain.

La composition de l'invention est notamment cosmétiquement et/ou dermatologiquement acceptable, c'est-à-dire compatible avec la peau, les ongles et les muqueuses. La composition contenant un ou plusieurs antagonistes de subtance P peut être appliquée sous les aisselles, les pieds, sur les mains, ainsi que sur toute autre zone cutanée du corps susceptible de transpirer. Elle peut notamment être utilisée dans le traitement de l'hypersudation comme le syndrôme de Frey ou pour le traitement des cuirs chevelus.

Pour qu'une substance soit reconnue comme un antagoniste de substance P, elle doit répondre à la caractéristique suivante : avoir une activité pharmacologique antagoniste de la substance P, c'est-à-dire induire une réponse pharmacologique cohérente dans au moins l'un des deux tests suivants :
- la substance antagoniste doit diminuer l'extravasation du plasma au travers de la paroi vasculaire induite par la capsaïcine ou par une stimulation nerveuse antidromique, ou bien
- la substance antagoniste doit provoquer une inhibition de la contraction des muscles lisses induites par l'administration de substance P.

En outre cet antagoniste de substance P peut avoir une affinité sélective pour les récepteurs NK1 des tachykinines.

Jusqu'à ce jour, les antagonistes de substance P étaient utilisés pour traiter les maladies indiquées ci-dessus ainsi que pour traiter les peaux sensibles. A cet effet, on peut se référer notamment aux documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569, GB-A-2216529, EP-A-360390, EP-A-429366, EP-A-430771, EP-A-499313, EP-A-514273, EP-A-514274, EP-A-514275, EP-A-514276, EP-A-520555, EP-A-528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, W0-A-93/01170, WO-A-93/06099, WO-A-93/09116, EP-A-522808 et WO-A-93/01165.

Personne jusqu'à ce jour n'avait établi un lien entre les antagonistes de la substance P et la transpiration.

Selon l'invention, on peut utiliser un ou plusieurs antagonistes de substance P choisis parmi les peptides, les composés comprenant au moins un hétérocycle, les composés azotés comprenant au moins un cycle benzènique, les sels de cations monovalents, divalents et trivalents, les extraits d'origine végétale ou animale et leurs mélanges.

L'antagoniste de substance P de l'invention peut être un peptide ou un dérivé azoté non peptidique, et plus précisément un composé comportant un hétérocycle azoté ou un atome d'azote lié directement ou indirectement à un cycle benzènique ou mieux un sel de certains cations monovalents, divalents et trivalents ou encore un mélange de ces antagonistes.

On peut utiliser dans l'invention par exemple comme peptide antagoniste de substance P le sendide et le spantide II.

Le sendide correspond à la formule :
Tyr D-Phe Phe D-His Leu Met NH₂
dans laquelle :
Tyr représente la tyrosine,
D-Phe représente la D-phénylalanine,
Phe représente la phénylalanine,
D-His représente la D-histidine,
Leu représente la leucine,
Met représente la méthionine.

Le spantide II correspond à la formule :
D-NicLys Pro 3-Pal Pro D-Cl₂Phe Asn D-Trp Phe D-Trp Leu Nle NH₂
dans laquelle :
D-NicLys représente.le nicotinate de D-lysine,
Pro représente la proline,
3-Pal représente la 3-pyridyl-alanine,
D-Cl₂Phe représente la D-dichlorophénylalanine,
Asn représente l'asparagine,
D-Trp représente le D-tryptophane,
Phe représente la phénylalanine,
Leu représente la leucine,
Nle représente la nor-leucine.

On peut également utiliser dans l'invention comme peptide antagoniste de substance P les peptides décrits dans les documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569 et GB-A-2216529.

Les antagonistes de substance P non peptidiques utilisables dans l'invention sont notamment des composés comprenant un hétéroatome lié directement ou indirectement à un cycle benzénique ou contenu dans un hétérocycle. En particulier cet hétéroatome est un atome d'oxygène, d'azote ou de soufre.

Comme composé hétérocyclique, on peut notamment utiliser dans l'invention ceux décrits dans les documents suivants : EP-A-360390, EP-A-429366, EP-A-430771, EP-A-499313, EP-A-514273, EP-A-514274, EP-A-514275, EP-A-514276, EP-A-520555, EP-A- 528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, WO-A-93/01170, WO-A-93/06099, WO-A-93/09116. En particulier, le composé comprenant au moins un hétérocycle azoté est un dérivé de 2-tricyclyl-2-amino-éthane, un dérivé de spirolactame, un dérivé de quinuclidine, un dérivé azacyclique, un dérivé d'aminopyrrolidine, un dérivé de pipéridine, un aminoazahétérocycle ou un dérivé d'isoindole.

Comme autres composés hétérocycliques, on peut citer les composés hétérocycliques oxygénés ou soufrés tels que les dérivés du furanne, les dérivés du benzofuranne, les dérivés du thiophène et les dérivés du benzothiophène, comportant éventuellement des substituants azotés, tels que les composés hétérocycliques décrits dans les documents US-A-4931459, US-A-4910317 et EP-A-299457, et plus spécialement les alcoxy- et/ou aryloxy- tétrazolylbenzofuranne-carboxamides ou les alcoxy- et/ou aryloxy- tétrazolylbenzothiophène-carboxamides.

Comme composés comportant un atome d'azote lié directement ou indirectement à un noyau benzénique, on peut citer ceux décrits dans les documents suivants : EP-A-522808 et WO-A-93/01165 et WO-A-93/10073.

Les sels de cations utilisables dans l'invention sont notamment les sels de strontium, de magnésium, de lanthanides de numéro atomique allant de 57 à 71, de cobalt, de nickel, de manganèse, de baryum, d'yttrium, de cuivre, d'étain, de rubidium, de lithium et de zinc.

Ces sels peuvent être par exemple des carbonates, des bicarbonates, des sulfates, des glycérophosphates, des borates, des chlorures, des nitrates, des acétates, des hydroxydes, des persulfates ainsi que des sels d'α-hydroxyacides (citrates, tartrates, lactates, malates) ou d'acides de fruits, ou encore des sels d'acides aminés (aspartate, arginate, glycocholate, fumarate) ou des sels d'acides gras (palmitate, oléate, caséinate, béhénate). De préférence le sel est choisi parmi le nitrate de strontium, de manganèse d'yttrium ou de magnésium, le borate de strontium, de manganèse, d'yttrium ou de magnésium, le chlorure de strontium, de manganèse ou de magnésium, le sulfate de magnésium, de manganèse ou de strontium. Encore plus préférentiellement ces sels sont le chlorure ou le nitrate de strontium.

Les antagonistes de substance P peuvent être synthétisés ou extraits de produits naturels (végétaux ou animaux). En particulier, on peut utiliser des extraits d'iridacée comme ceux d'Iris pallida ou de bactérie filamenteuse.

Dans les compositions selon l'invention, l'antagoniste de substance P est utilisé de préférence en une quantité allant de 0,000001 à 20 % du poids total de la composition, et en particulier en une quantité représentant de 0,0001 à 15 % du poids total de la composition . Pour des sels on utilise de préférence une quantité représentant de 0,01 à 20 % du poids total de la composition, et en particulier en une quantité représentant de 0,1 à 15 % du poids total de la composition et mieux de 0,5 à 8 %. Pour les dérivés peptidiques et non peptidiques à hététoatome, les quantités utilisées sont avantageusement de 0,000001 à 10 % du poids total de la composition et mieux de 0,0001 à 5 %.

Les compositions selon l'invention peuvent se présenter sous toutes les formes galéniques normalement utilisées pour une application topique sur la peau et/ou le cuir chevelu, notamment des solutions aqueuses, alcooliques ou hydroalcooliques ou dispersions du type lotion ou sérum, des émulsions de consistance liquide ou semi-liquide du type lait, obtenues par dispersion d'une phase grasse dans une phase aqueuse (H/E) ou inversement (E/H), ou des suspensions ou émulsions de consistance molle du type crème, mousse ou gel, ou encore des microgranulés, ou des dispersions vésiculaires de type ionique et/ou non ionique. Ces compositions peuvent, en outre, se présenter sous forme de stick (produit solide) ou de produit mis sous pression par un agent propulseur (aérosol). Elles sont, de plus, préparées selon les méthodes usuelles.

Les quantités des différents constituants des compositions selon l'invention sont celles classiquement utilisées dans les domaines considérés.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition de l'invention est une solution ou un gel huileux, la phase grasse peut représenter plus de 90 % du poids total de la composition.

De façon connue, la composition cosmétique et/ou dermatologique de l'invention peut contenir également des adjuvants habituels dans les domaines concernés, tels que les gélifiants hydrophiles ou lipophiles, les actifs hydrophiles ou lipophiles, les conservateurs, les antioxydants, les solvants, les parfums, les charges (talc), les filtres, les bactéricides, les absorbeurs d'odeur et les matières colorantes. Les quantités de ces différents adjuvants sont celles classiquement utilisées dans le domaine cosmétique, et par exemple de 0,01 % à 20 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclométhicone) et les huiles fluorées (perfluoropolyéthers). On peut ajouter à ces huiles des alcools gras et des acides gras (acide stéarique) ainsi que des cires (d'abeilles, de paraffine, de carnauba).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon, des extraits bactériens ou végétaux comme ceux d'Aloe Vera ainsi que les hydroxyacides (citrique, lactique, glycolique, tartrique et de manière générale les acides de fruits).

Comme actifs lipophiles, on peut utiliser le rétinol (vitamine A) et ses dérivés, les acides gras essentiels, les céramides, les huiles essentielles, l'acide salicylique et ses dérivés (n-octanoyl-5-salicylique). En particulier, les acides salicylique, lactique, acétique servent notamment d'antiseptiques.

Comme autres actifs utilisables dans l'invention, on peut citer à titre d'exemple :
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents anti-radicaux libres, tels que l'alpha-tocophérol (vitamine E) ou ses esters, les superoxyde dismutases, certains chélatants de métaux ou l'acide ascorbique et ses esters ;
- les antiséborrhéiques tels que la progestérone .

De façon avantageuse, les antagonistes de substance P de l'invention peuvent être associés à des actifs à effet secondaire irritant utilisés couramment dans le domaine des déodorants. La présence d'un antagoniste de substance P dans une composition déodorante contenant un actif ayant un effet irritant permet d'atténuer fortement, voire de supprimer cet effet irritant. Ainsi, il est possible selon l'invention d'utiliser, en faible quantité, les agents antitranspirants de l'art antérieur, associés avec un ou plusieurs agents antitranspirants de l'invention.

La présente invention a en outre pour objet un procédé de traitement cosmétique et/ou dermatologique, caractérisé par le fait que l'on applique sur la peau une composition telle que décrite ci-dessus contenant, comme agent antitranspirant, au moins un antagoniste de substance P dans un milieu cosmétiquement ou dermatologiquement acceptable.

Le procédé de traitement cosmétique de l'invention peut être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits.

Les exemples suivants illustrent l'invention. Dans ces exemples, les proportions indiquées sont des pourcentages en poids.

### Exemple 1 : Crème antitranspirante aqueuse (huile/eau) (nomenclature CTFA)

| *Phase A : grasse* | |
|---|---|
| - Cetearyl alcohol (corps gras, gélifiant) | 4 % |
| - Glyceryl stearate (corps gras, gélifiant) | 2,5 % |
| - Steareth-25 (émulsionnant) | 1,05 % |
| - Stearyl alcohol (corps gras, gélifiant) | 1,05 % |
| - Ceteareth-33 (émulsionnant) | 1 % |
| - Dimethicone | 1 % |
| - Ceteth-20 (émulsionnant) | 0,4 % |
| - Conservateur qs | |

| *Phase B : aqueuse* | |
|---|---|
| - Eau qsp | 100 % |
| - Chlorure de strontium (antitranspirant) | 13 % |
| - Conservateur qs | |

| *Phase C: actif* | |
|---|---|
| - Parfum qs | |
| - Bactéricide qs | |

| *Phase D:* | |
|---|---|
| - Talc | 1 % |

**Mode opératoire** : Chauffer la phase B à 70-80 °C, sous agitation. Chauffer la phase A à 70-80 °C. Lorsque les deux phases sont bien homogènes, incorporer A dans B, sous agitation forte pendant 10 min. Refroidir sous agitation plus faible. A 45 °C, ajouter la phase C prémélangée. Agiter fortement pendant 5 min. Refroidir sous agitation plus faible. Vers 30 °C, incorporer lentement la phase D. Poursuivre le refroidissement jusqu'à la température ambiante.

On obtient une crème souple et douce, à effet poudré à l'étalement procurant une protection efficace contre la transpiration.

### Exemple 2 : Crème anhydre antitranspirante (nomenclature CTFA)

| *Phase A :* | |
|---|---|
| - Stearalkonium hectorite (gélifiant) | 4,2 % |
| - Propylene carbonate (agent de mise en suspension) | 1,4 % |
| - Caprylic/Capric Triglycerides | 29,4 % |
| - Dimethicone | 5 % |
| - Isohexadecane (solvant) qsp | 100 % |

| *Phase B:* | |
|---|---|
| - Chlorure de baryum (antitranspirant) | 15 % |
| - Talc | 1 % |

| *Phase C:* | |
|---|---|
| - Parfum qs | |

| *Phase D:* | |
|---|---|
| - Talc | 6 % |

**Mode opératoire** : Mélanger les composés de la phase A à température ambiante. Incorporer ensuite la phase C, toujours à température ambiante. Lorsque tout est homogène, incorporer B et D lentement.

La crème obtenue est particulièrement douce, non grasse, non collante, et procure par son impact sec, une sensation de plus grande sécurité contre l'humidité axillaire.

### Exemple 3 : Aérosol (nomenclature CTFA)

| *Phase A:* | |
|---|---|
| - Cyclomethicone qsp | 100 % |
| - Quaternium-18 bentonite | 3 % |
| - Triethylcitrate | 7 % |
| - Dimethiconol | 1,4 % |
| - Isopropyl palmitate | 6 % |

| *Phase B:* | |
|---|---|
| - Nitrate de strontium (antitranspirant) | 15 % |

| *Phase C:* | |
|---|---|
| - Talc | 1,5 % |

**Mode opératoire** : Mélanger les ingrédients de la phase A à froid. Ajouter lentement le chlorohydrate d'aluminium, puis le talc. Mélanger vigoureusement pour obtenir une suspension homogène. Cette dernière peut ensuite être pressurisée de la façon suivante : 13 % de jus, 87 % de gaz comprimé ou liquéfié.

Le jus délivré laisse un impact doux, poudré sur la peau assurant la protection contre la transpiration.

### Exemple 4 : Poudre compactée

| | |
|---|---|
| - Chlorure de magnésium (antitranspirant) | 5 % |
| - Talc qsp | 100 % |
| - Kaolin | 10 % |
| - Chlorure de strontium (antitranspirant) | 15 % |
| - Parfum | 0,30 % |

### Exemple 5 : Stick

| | |
|---|---|
| - Chlorure de baryum (antitranspirant) | 10 % |
| - Alcool stéarylique | 24 % |
| - Distéarate de PEG-400 | 6 % |
| - Cire hydrocarbonnée | 4 % |
| - Cyclométhicone | 46 % |

## Revendications

1. Utilisation d'au moins un antagoniste de substance P dans ou pour la préparation d'une composition topique comme agent antitranspirant .

2. Utilisation d'au moins un antagoniste de substance P dans ou pour la préparation d'une composition cosmétique ou dermatologique, pour prévenir et/ou lutter contre la transpiration cutanée.

3. Utilisation selon la revendication 1 ou 2, caractérisée en ce que l'antagoniste de substance P est choisi parmi les substances assurant une diminution de l'extravasation du plasma au travers de la paroi vasculaire induite par la capsaïcine ou par une stimulation nerveuse antidromique et les substances provoquant une inhibition de la contraction des muscles lisses induites par l'administration de substance P.

4. Utilisation selon l'une quelconque des revendications précédentes, caractérisée en ce que les antagonistes sont choisis parmi les peptides, les composés comprenant au moins un hétérocycle, les composés azotés comprenant au moins un cycle benzènique, les sels de cations monovalents, divalents et trivalents, les extraits d'origine végétale ou animale et leurs mélanges.

5. Utilisation selon la revendication précédente, caractérisée en ce que le peptide est le sendide ou le spantide II.

6. Utilisation selon la revendication 4, caractérisée en ce que le composé comprenant au moins un hétérocycle est un composé hétérocyclique azoté choisi parmi les dérivés de 2-tricyclyl-2-amino-éthane, les dérivés de spirolactame, les dérivés de quinuclidine, les dérivés azacycliques, les dérivés d'aminopyrrolidine, les dérivés de pipéridine, les aminoazahétérocycles, les dérivés d'isoindole.

7. Utilisation selon la revendication 4, caractérisée en ce que le composé comprenant au moins un hétérocycle est un composé hétérocyclique oxygéné ou soufré choisi parmi les dérivés du furanne, les dérivés du benzofuranne, les dérivés du thiophène et les dérivés du benzothiophène, et notamment les tétrazolyl-benzofuranne-carboxamides ou les tétrazolyl-benzothiophène-carboxamides.

8. Utilisation selon la revendication 4, caractérisée en ce que le sel est choisi parmi les chlorures, carbonates, bicarbonates, borates, nitrates, hydroxydes, sulfates, persulfates, les sels d'acides de fruits, les sels d'acides aminés et les sels d'acides gras de baryum, magnésium, strontium, d'yttrium, de gadolinium, de manganèse, de zinc et/ou de cobalt.

9. Utilisation selon la revendication 4, caractérisée en ce que le sel est choisi parmi les sels de strontium.

10. Utilisation selon l'une quelconque des revendications précédentes, caractérisée en ce que l'antagoniste de substance P est choisi parmi le chlorure ou le nitrate de strontium, les extraits d'Iris, et les extraits de bactérie filamenteuse.

11. Utilisation selon l'une quelconque des revendications précédentes, caractérisée en ce que l'antagoniste de substance P est utilisé en une quantité allant de 0,000001 à 20 % en poids par rapport au poids total de la composition.

12. Utilisation selon l'une quelconque des revendications précédentes, caractérisée en ce que l'antagoniste de substance P est utilisé en une quantité allant de 0,0001 à 15 % en poids par rapport au poids total de la composition.

13. Utilisation selon l'une quelconque des revendications précédentes, caractérisée en ce que la composition est une solution aqueuse, alcoolique ou hydroalcoolique, une émulsion eau-dans-huile, une émulsion huile-dans-eau, une microémulsion, un gel aqueux, un gel anhydre, un sérum, une dispersion de vésicules, un produit solide.

14. Utilisation selon l'une quelconque des revendications précédentes, caractérisée en ce que la composition contient au moins un agent choisi parmi les agents antibactériens, antiparasitaires, antifongiques, anti-inflammatoires, anesthésiques, anti-radicaux libres, antiséborrhéiques, absorbeurs d'odeur, parfums, antiseptiques.

15. Procédé de traitement cosmétique destiné à prévenir et/ou lutter contre la transpiration cutanée, caractérisé en ce que l'on applique sur la peau une composition contenant, comme agent antitranspirant, au moins un antagoniste de substance P dans un milieu cosmétiquement acceptable.

16. Procédé selon la revendication 15, caractérisé en ce que l'antagoniste de substance P est choisi parmi les peptides, les composés comprenant au moins un hétérocycle, les composés azotés comprenant au moins un cycle benzènique, les sels de cations monovalents, divalents et trivalents, les extraits d'origine végétale ou animale et leurs mélanges.

17. Procédé selon les revendications 16, caractérisé en ce que l'antagoniste de substance P est choisi parmi les chlorures, carbonates, bicarbonates, borates, nitrates, hydroxydes, sulfates, persulfates, les sels d'acides de fruits, les sels d'acides aminés et les sels d'acides gras de baryum, magnésium, strontium, d'yttrium, de gadolinium, de manganèse, de zinc et/ou de cobalt.

18. Procédé selon l'une quelconque des revendications 15 à 17, caractérisé en ce que l'antagoniste de substance P est utilisé en une quantité allant de 0,0001 à 15 % en poids par rapport au poids total de la composition.

## Claims

1. Use of at least one substance P antagonist in or for the preparation of a topical composition as antiperspirant agent.

2. Use of at least one substance P antagonist in or for the preparation of a cosmetic or dermatological composition for preventing and/or controlling cutaneous perspiration.

3. Use according to Claim 1 or 2, characterized in that the substance P antagonist is chosen from substances providing a decrease in the extravasation of the plasma through the vascular wall induced by capsaicin or by an antidromic nerve stimulation and substances causing inhibition of the contraction of the smooth muscles induced by the administration of substance P.

4. Use according to any one of the preceding claims, characterized in that the antagonists are chosen from peptides, compounds comprising at least one heterocycle, nitrogenous compounds comprising at least one benzene ring, salts of monovalent, divalent and trivalent cations, extracts of plant or animal origin and their mixtures.

5. Use according to the preceding claim, characterized in that the peptide is sendide or spantide II.

6. Use according to Claim 4, characterized in that the compound comprising at least one heterocycle is a nitrogenous heterocyclic compound chosen from 2-tricyclyl-2-aminoethane derivatives, spirolactam derivatives, quinuclidine derivatives, azacyclic derivatives, aminopyrrolidine derivatives, piperidine derivatives, aminoazaheterocycles or isoindole derivatives.

7. Use according to Claim 4, characterized in that the compound comprising at least one heterocycle is an oxygen-containing or sulphur-containing heterocyclic compound chosen from furan derivatives, benzofuran derivatives, thiophene derivatives and benzothiophene derivatives and in particular tetrazolylbenzofurancarboxamides or tetrazolylbenzothiophenecarboxamides.

8. Use according to Claim 4, characterized in that the salt is chosen from barium, magnesium, strontium, yttrium, gadolinium, manganese, zinc and/or cobalt chlorides, carbonates, bicarbonates, borates, nitrates, hydroxides, sulphates, persulphates, salts of fruit acids, salts of amino acids and salts of fatty acids.

9. Use according to Claim 4, characterized in that the salt is chosen from strontium salts.

10. Use according to any one of the preceding claims, characterized in that the substance P antagonist is chosen from strontium chloride or nitrate, Iris extracts and filamentous bacterium extracts.

11. Use according to any one of the preceding claims, characterized in that the substance P antagonist is used in an amount ranging from 0.000001 to 20 % by weight with respect to the total weight of the composition.

12. Use according to any one of the preceding claims, characterized in that the substance P antagonist is used in an amount ranging from 0.0001 to 15 % by weight with respect to the total weight of the composition.

13. Use according to any one of the preceding claims, characterized in that the composition is an aqueous, alcoholic or aqueous/alcoholic solution, a water-in-oil emulsion, an oil-in-water emulsion, a microemulsion, an aqueous gel, an anhydrous gel, a serum, a vesicular dispersion or a solid product.

14. Use according to any one of the preceding claims, characterized in that the composition contains at least one agent chosen from antibacterial agents, agents for combating parasites, antifungal agents, antiinflammatory agents, anaesthetic agents, agents for combating free radicals, antiseborrhoeic agents, odour absorbers, fragrances or antiseptics.

15. Cosmetic treatment process intended to prevent and/or to control cutaneous perspiration, characterized in that a composition containing, as antiperspirant agent, at least one substance P antagonist in a cosmetically acceptable medium is applied on the skin.

16. Process according to Claim 15, characterized in that the substance P antagonist is chosen from peptides, compounds comprising at least one heterocycle, nitrogenous compounds comprising at least one benzene ring, salts of monovalent, divalent and trivalent cations, extracts of plant or animal origin and their mixtures.

17. Process according to Claim 16, characterized in that the substance P antagonist is chosen from barium, magnesium, strontium, yttrium, gadolinium, manganese, zinc and/or cobalt chlorides, carbonates, bicarbonates, borates, nitrates, hydroxides, sulphates, persulphates, salts of fruit acids, salts of amino acids and salts of fatty acids.

18. Process according to any one of Claims 15 to 17, characterized in that the substance P antagonist is used in an amount ranging from 0.0001 to 15 % by weight with respect to the total weight of the composition.

## Patentansprüche

1. Verwendung von mindestens einem Antagonisten der Substanz P in einer topischen Zusammensetzung als Antitranspirationsmittel oder zur Herstellung dieser Zusammensetzung.

2. Verwendung mindestens eines Antagonisten der Substanz P in einer kosmetischen oder dermatologischen Zusammensetzung zur Vorbeugung und/oder zur Bekämpfung der Hauttranspiration oder zur Herstellung einer derartigen Zusammensetzung.

3. Verwendung nach Anspruch 1 oder 2, dadurch gekennzeichnet, daß der Antagonist der Substanz P unter den Substanzen, die eine Verringerung der Extravasation von Plasma durch die Gefäßwand, die durch Capsaicin oder durch eine gegenläufige Nervenstimulation induziert wird, gewährleisten, und den Substanzen, die eine Hemmung der Kontraktion der glatten Muskulatur, die durch die Verabreichung der Substanz P induziert wird, hervorrufen, ausgewählt ist.

4. Verwendung nach einem der vorstehenden Ansprüche, dadurch gekennzeichnet, daß die Antagonisten unter Peptiden, Verbindungen mit mindestens einem Heterocyclus, stickstoffhaltigen Verbindungen mit mindestens einem Benzolring, Salzen von einwertigen, zweiwertigen und dreiwertigen Kationen, Extrakten pflanzlichen oder tierischen Ursprungs und Gemischen davon ausgewählt sind.

5. Verwendung nach dem vorstehenden Anspruch, dadurch gekennzeichnet, daß es sich beim Peptid um Sendid oder Spantid II handelt.

6. Verwendung nach Anspruch 4, dadurch gekennzeichnet, daß es sich bei der Verbindung, die mindestens einen Heterocyclus enthält, um einen stickstoffhaltigen Heterocyclus handelt, der unter den Derivaten von 2-Tricyclyl-2-amino-ethan, den Derivaten von Spirolactam, den Derivaten von Chinuclidin, den azacyclischen Derivaten, den Derivaten von Aminopyrrolidin, den Derivaten von Piperidin, den Aminoazaheterocyclen und den Derivaten von Isoindol ausgewählt ist.

7. Verwendung nach Anspruch 4, dadurch gekennzeichnet, daß es sich bei der Verbindung, die mindestens einen Heterocyclus enthält, um eine Sauerstoff oder Schwefel enthaltende heterocyclische Verbindung handelt, die unter den Derivaten von Furan, den Derivaten von Benzofuran, den Derivaten von Thiophen und den Derivaten von Benzothiophen und insbesondere unter Tetrazolylbenzofurancarboxamiden oder Tetrazolylbenzothiophencarboxamiden ausgewählt ist.

8. Verwendung nach Anspruch 4, dadurch gekennzeichnet, daß das Salz unter den Chloriden, Carbonaten, Bicarbonaten, Boraten, Nitraten, Hydroxiden, Sulfaten, Persulfaten, Salzen von Fruchtsäuren, Salzen von Aminosäuren und Salzen von Fettsäuren von Barium, Magnesium, Strontium, Yttrium, Gadolinium, Mangan, Zink und/oder Cobalt ausgewählt ist.

9. Verwendung nach Anspruch 4, dadurch gekennzeichnet, daß das Salz unter den Salzen von Strontium ausgewählt ist.

10. Verwendung nach einem der vorstehenden Ansprüche, dadurch gekennzeichnet, daß der Antagonist der Substanz P unter dem Chlorid oder Nitrat von Strontium, Extrakten von Iris und Extrakten filamentöser Bakterien ausgewählt ist.

11. Verwendung nach einem beliebigen der vorstehenden Ansprüche, dadurch gekennzeichnet, daß der Antagonist der Substanz P in einer Menge von 0,000001 bis 20 Gew.-%, bezogen auf das Gesamtgewicht der Zusammensetzung, verwendet wird.

12. Verwendung nach einem der vorstehenden Ansprüche, dadurch gekennzeichnet, daß der Antagonist der Substanz P in einer Menge von 0,0001 bis 15 Gew.-%, bezogen auf das Gesamtgewicht der Zusammensetzung, verwendet wird.

13. Verwendung nach einem der vorstehenden Ansprüche, dadurch gekennzeichnet, daß es sich bei der Zusammensetzung um eine wäßrige, alkoholische oder wäßrig-alkoholische Lösung, eine Wasser-in-Öl-Emulsion, eine Öl-in-Wasser-Emulsion, eine Mikroemulsion, ein wäßriges Gel, ein wasserfreies Gel, ein Serum, eine Dispersion von Vesikeln oder ein festes Produkt handelt.

14. Verwendung nach einem der vorstehenden Ansprüche, dadurch gekennzeichnet, daß die Zusammensetzung mindestens ein Mittel enthält, daß unter antibakteriellen Mitteln, antiparasitären Mitteln, Pilzbekämpfungsmitteln, entzündungshemmenden Mitteln, anästhetischen Mitteln, Mitteln gegen freie Radikale, Mitteln gegen Seborrhoe, geruchsabsorbierenden Mitteln, Parfums oder antiseptischen Mitteln ausgewählt ist.

15. Verfahren zur kosmetischen Behandlung zur Verhinderung und/oder zur Bekämpfung von Hauttranspiration, dadurch gekennzeichnet, daß man auf die Haut eine Zusammensetzung aufbringt, die als Antitranspirationsmittel mindestens einen Antagonisten der Substanz P in einem kosmetisch verträglichen Medium enthält.

16. Verfahren nach Anspruch 15, dadurch gekennzeichnet, daß die Antagonisten unter Peptiden, Verbindungen mit mindestens einem Heterocyclus, stickstoffhaltigen Verbindungen mit mindestens einem Benzolring, Salzen von einwertigen, zweiwertigen und dreiwertigen Kationen, Extrakten pflanzlichen oder tierischen Ursprungs und Gemischen davon ausgewählt sind.

17. Verfahren nach Anspruch 16, dadurch gekennzeichnet, daß der Antagonist der Substanz unter den Chloriden, Carbonaten, Bicarbonaten, Boraten, Nitraten, Hydroxiden, Sulfaten, Persulfaten, Salzen von Fruchtsäuren, Salzen von Aminosäuren und Salzen von Fettsäuren von Barium, Magnesium, Strontium, Yttrium, Gadolinium, Mangan, Zink und/oder Cobalt ausgewählt ist.

18. Verfahren nach Anspruch 15 bis 17, dadurch gekennzeichnet, daß der Antagonist der Substanz P in einer Menge von 0,0001 bis 15 Gew.-%, bezogen auf das Gesamtgewicht der Zusammensetzung, verwendet wird.
